# EUROPEAN PATENT APPLICATION

(11) **EP 2 042 119 A1**
(43) Date of publication of application: **01.04.2009**
(21) Application number: 08253033.8
(22) Date of filing: 15.09.2008
(51) Int. Cl.: A61B 18/14

(54) **A catheter assembly**

(30) Priority: 28.09.2007 US 995791 P
(71) Applicant: CathRx Ltd, Eveleigh, New South Wales 1430 (AU)
(72) Inventor: Anderson, Neil Lawrence, Roseville New South Wales 2069 (AU)
(74) Representative: Nettleton, John Victor

(57) **Abstract**

A catheter sheath 10 includes an elongate element 12 defining a primary lumen 18 and a plurality of secondary lumens 20 arranged in spaced relationship about the primary lumen 18. A plurality of discrete members 14 are defined by the elongate element 12, the discrete members 14 being displaceable between a first position extending parallel to a longitudinal axis of the elongate element 12 and a second position in which the discrete members 14 extend transversely to the longitudinal axis of the elongate element 12. At least one electrode 22 is carried by at least one of the discrete members 14. Electrical conductors 24 for the at least one electrode 22 are arranged in the primary lumen 18, the primary lumen 18 being shaped to accommodate the conductors 24.

## Description

### Cross-Reference to Related Applications

The present application claims priority from United States of America Provisional Patent Application No 60/995,791 filed on September 28, 2007, the contents of which are incorporated herein by reference in their entirety.

### Field

This invention relates generally to the field of catheters and, more particularly, to a catheter sheath for a catheter assembly and to a catheter assembly including the catheter sheath.

### Background

In the field of heat treatment of tissue, it is desirable if the device heating the tissue is in contact only with the tissue being treated and that contact with surrounding tissue or bodily fluids is minimised. This reduces the power required to heat the tissue and also minimises unnecessary damage to other tissue, structures or fluid.

In addition, it is often necessary to overcome tissue irregularities at a site in a patient's body being heat treated. An example where a site in a patient's body is subjected to heat treatment is in the treatment of heart arrhythmias where tissue is ablated in an effort to cure the arrhythmia. The tissue is ablated to create a lesion to block the electrical impulses causing the arrhythmia. To ensure that a lesion of adequate depth is formed, it is desirable that the ablating electrode make good contact with the tissue. Other examples of the use of heat treatment at a site in a patient's body include treatment of Parkinson's disease, tumour ablation, endometriosis and pain management.

Still further, in the treatment of arrhythmias, it may be necessary to ablate over a reasonably wide area in an attempt to cure the arrhythmia. It would be beneficial to be able to obtain such larger ablated areas with minimum manipulation of the catheter when in position at the site to be treated.

There is therefore a need for a catheter sheath and a catheter assembly which meets these needs. Such a catheter sheath and catheter assembly could also be useful in other applications, for example, pacing, sensing or defibrillation.

### Summary

According to a first aspect of the invention, there is provided a catheter sheath which includes
an elongate element defining a primary lumen and a plurality of secondary lumens arranged in spaced relationship about the primary lumen;
a plurality of discrete members defined by the elongate element, the discrete members being displaceable between a first position extending parallel to a longitudinal axis of the elongate element and a second position in which the discrete members extend transversely to the longitudinal axis of the elongate element;
at least one electrode carried by at least one of the discrete members; and
electrical conductors for the at least one electrode arranged in the primary lumen, the primary lumen being shaped to accommodate the conductors.

An inner surface of a wall of the elongate element defining the primary lumen may be recessed to define at least one axially extending channel in which the conductors are receivable. Preferably, each discrete member carries at least one electrode and a plurality of circumferentially spaced channels may be defined in the inner surface of the wall of the elongate element, each discrete member having a channel associated with it.

The at least one electrode of each discrete member may be arranged on an operatively inner surface of its associated discrete member. By "operatively inner surface" means that surface of the discrete member which, when it is in its first position, faces an opposed discrete member.

The discrete members may be defined by the elongate element. More particularly, the discrete members may be defined by longitudinally extending slits at least at the distal end of the elongate element. The catheter sheath may include a sleeve with the elongate element being mounted on a distal end of the sleeve. In an embodiment, the slits may extend the full length of the elongate element. The discrete members may be retained in position relative to each other by being attached to the distal end of the sleeve. It will be appreciated that the discrete members could be attached within the distal end of the sleeve or to an external surface of the distal end of the sleeve.

A control element, in the form of a pull wire, may be received in each secondary lumen, each control element being connected to one of the discrete members and the control element controlling movement of its associated discrete member at least from its first position to its second position.

The catheter sheath may include an urging means associated with each discrete member for urging the discrete member from its second position to its first position. Each urging means may be a resiliently flexible component having at least a portion inwardly of a centre plane, or plane of bending, of its associated discrete member. Thus, each flexible component may be attached to an inner surface of the discrete member, i.e. in the primary lumen or, instead, each flexible component may be arranged in the secondary lumen of its associated discrete member inwardly of the control element.

According to a second aspect of the invention, there is provided a catheter assembly which includes
a handle having a proximal end and a distal end;
a catheter sheath as described above attached to a distal end of the handle; and
a control mechanism carried by the handle, the control elements of the catheter sheath being connected to the control mechanism.

The assembly may include a stylet received in the primary lumen of the elongate element of the catheter sheath. Preferably, the stylet is a steerable stylet for effecting steering of the catheter sheath, in use.

The handle may include a steering control mechanism to which the stylet is connected for effecting steering of the stylet.

A distal end of the stylet may carry an end electrode. In an embodiment, the end electrode may be a dome electrode.

The end electrode may be arranged to lie substantially in the same plane as the at least one electrode of the at least one discrete member when that discrete member is in its second position. For this purpose, the stylet and the catheter sheath may be axially displaceable with respect to each other so that, when the discrete members of the elongate element of the catheter sheath are in their second position, the end electrode can be brought into substantially the same plane as the at least one electrode of the at least one discrete element.

In another embodiment, the end electrode may be a needle electrode.

The control mechanism may include a plurality of control members, each control member being connected to one of the control elements of the catheter sheath so that movement of each discrete member can be effected independently of the remaining discrete members. Each control member may be in the form of a slide which is slidable axially in the handle. The control mechanism may include a connector, in the form of a collar, for interconnecting the control members to effect simultaneous control of the discrete members of the catheter sheath.

### Brief Description of Drawings

Fig. 1 shows a three dimensional view of a first embodiment of a catheter sheath;
Fig. 2 shows, on an enlarged scale, an end view of the catheter sheath of Fig. 1;
Fig. 3 shows a sectional side view of the catheter sheath taken along line III-III in Fig. 2;
Fig. 4 shows a three dimensional view of a second embodiment of a catheter sheath;
Fig. 5 shows, on an enlarged scale, an end view of the catheter sheath of Fig. 4;
Fig. 6 shows a sectional side view of the catheter sheath taken along line VI-VI in Fig. 5;
Fig. 7 shows a side view of an embodiment of a catheter assembly;
Fig. 8 shows a sectional side view of the catheter assembly taken along line VIII-VIII in Fig. 7;
Fig. 9 shows, on an enlarged scale, the part of the catheter of Fig. 8 encircled by Circle 'A';
Fig. 10 shows a side view of the catheter assembly with a connector of a control mechanism of the catheter omitted;
Fig. 11 shows a three dimensional view of a distal part of the catheter assembly with discrete members of a catheter sheath of the assembly in an open position;
Fig. 12 shows a sectional side view of the distal part of the catheter assembly with the discrete members of the catheter sheath of the assembly in the open position; and
Fig. 13 shows a three dimensional view of a distal part of one of the discrete members of the catheter sheath.

### Detailed Description of Exemplary Embodiments

In the drawings, reference numeral 10 generally designates an embodiment of a catheter sheath. The catheter sheath 10 comprises an elongate element 12. The catheter sheath 10 further includes a plurality of discrete members, or petals, 14 defined by, and arranged at a distal end of, the elongate element 12. The petals 14 are formed by slits 16 cut in the distal end of the elongate element 12.

In another embodiment, the slits 16 could extend the full length of the elongate element 12, with the elongate element 12 then being supported by a sleeve (not shown). The elongate element 12 could be attached to an inner surface or an outer surface of the sleeve to be supported by the sleeve.

The elongate element 12 is in the form of a tube and defines a central lumen 18. The central lumen 18 is a primary lumen and is surrounded by a plurality of secondary lumens 20. Each petal 14 has a secondary lumen 20 associated with it.

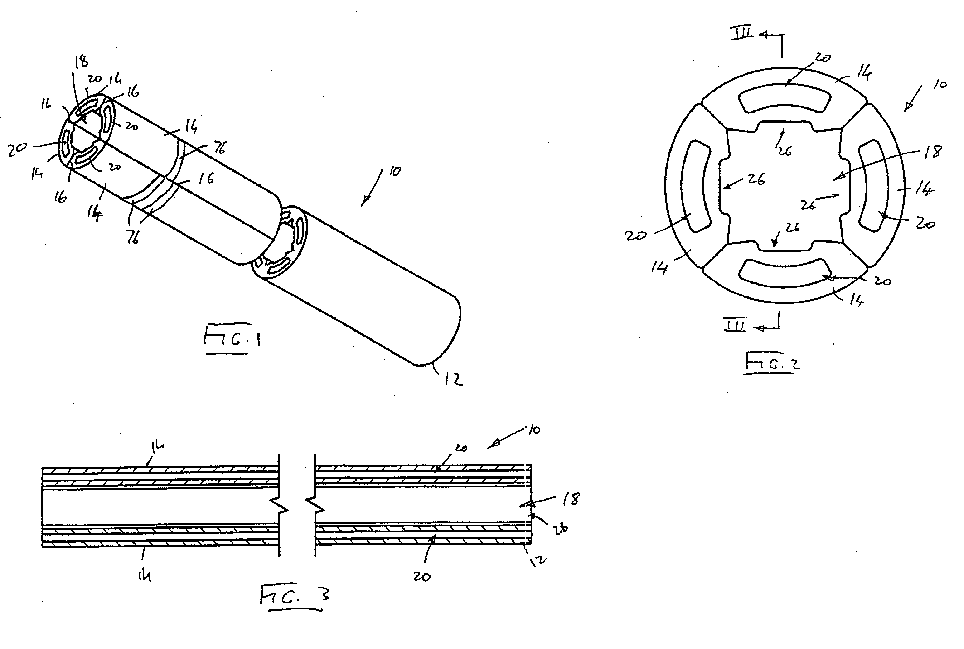

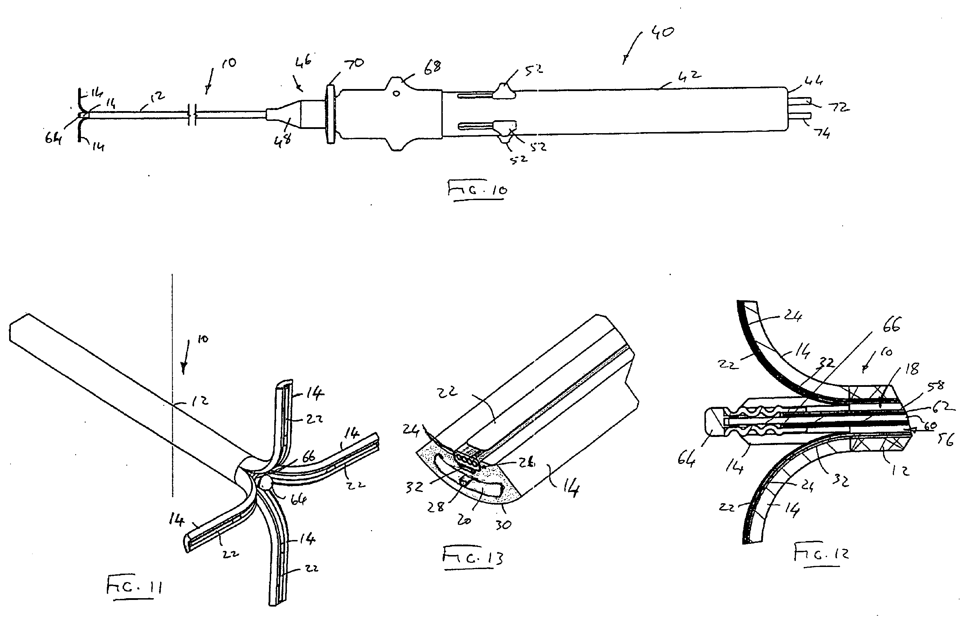

As shown most clearly in Figs. 11-13 of the drawings, an operatively inner surface of each petal 14 carries an electrode 22. The electrode 22 is an elongate electrode extending axially when the petals 14 are in their first, closed position as shown in Figs. 1-6 of the drawings. As will be described in greater detail below, the petals 14 of the catheter sheath 10 are displaceable from the first position to a second position as shown, for example, in Figs. 11 and 12 of the drawings. In this second position, the petals 14 lie transversely to a longitudinal axis of the elongate element 12 and, more particularly, lies in a plane substantially normal to the longitudinal axis of the elongate element 12.

Each electrode 22 has a group of conductors associated with it. As illustrated most clearly in Fig. 13 of the drawings, the conductors are grouped in ribbon cable 24. The primary lumen 18 of the elongate element 12 is shaped to accommodate the ribbon cable 24. The ribbon cable 24 comprises four conductors, two for the delivery of radiofrequency (RF) energy and a copper/Constantin pair for a thermocouple to monitor temperature at the electrode 22 during RF ablation by the electrode 22. It will be appreciated that, instead of a thermocouple, a thermistor or other heat sensing device could be used for temperature monitoring and the pair of conductors of the ribbon cable 24 for the heat sensing device would be appropriate for that heat sensing device.

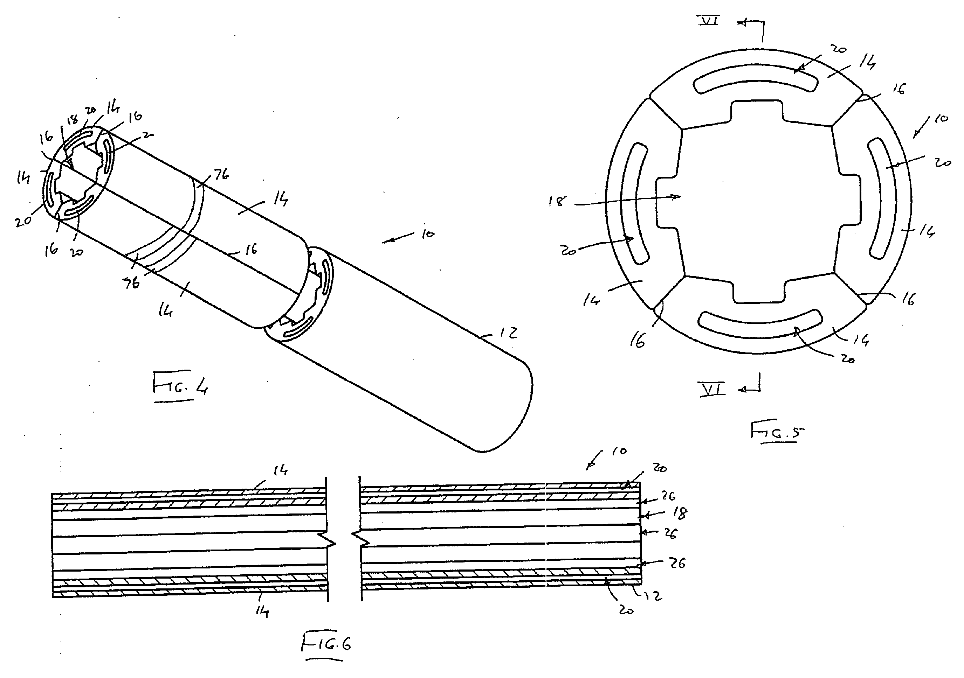

Spaced channels, or recesses, 26 (Figs 2 and 5) are formed in an inner surface of a wall of the elongate element 12 defining the primary lumen 18. A ribbon cable 24 is received in each channel 26. Thus, each petal 14 has a recess 26 associated with it.

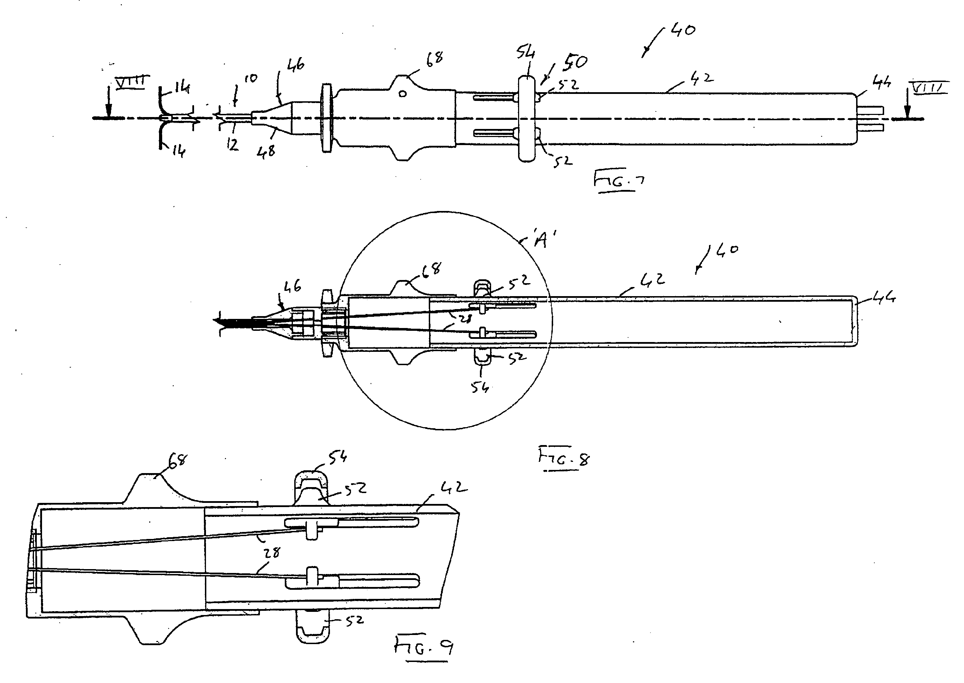

The catheter sheath 10 includes a control element in the form of a pull wire 28 (Figs. 8 and 9) received in each secondary lumen 20. It is to be noted that, as shown in Fig. 13 of the drawings, the pull wire 28 is on that side of the secondary lumen 20 closer to an outer surface 30 of the petal 14 so that, effectively, the pull wire 28 lies outwardly of a plane of bending of the petal 14.

The catheter sheath 10 is of a plastics material. The plastics material may be a medical grade plastics material such as that sold under the trade name PEBAX or it may be an elastomeric material such as a suitable silicone composition. In the latter case, in the event that the material is too flexible to allow the petals 14 to adopt their first position after they have been in their second position, the catheter sheath 10 includes an urging means in the form of a strip 32 (Fig. 13) of a resiliently flexible material. More particularly, the strip 32 is of a shape memory alloy material such as Nitinol.

In the embodiment illustrated in Fig. 13 of the drawings, the Nitinol strip 32 is arranged beneath the ribbon cable 24 in the recess 26 of the primary lumen 18. It will, however, be appreciated that each Nitinol strip 32 could be arranged in the associated secondary lumen 20 inwardly of the pull wire 28 and inwardly of the plane of bending of the petal 14.

The embodiment of the catheter sheath 10 shown in Figs. 4 to 6 of the drawings is similar to that shown in Figs. 1 to 3 of the drawings. Thus, with reference to Figs. 1 to 3 of the drawings, like reference numerals refer to like parts, unless otherwise specified. The difference between the two embodiments is in the dimensions of the primary lumen 18, the secondary lumens 20 and the recesses 26.

In Figs. 7-10 of the drawings, an embodiment of a catheter assembly is designated generally by the reference numeral 40. The catheter assembly 40 comprises a handle 42 having a proximal end 44 and a distal end 46. The catheter sheath 10 is attached via a strain relief 48 to the distal end 46 of the handle 44.

The catheter assembly 40 includes a control mechanism 50 for controlling displacement of the petals 14 of the elongate element 12 of the catheter sheath 10 between the first position as shown in Figs. 1-6 of the drawings and the position as shown, for example, in Figs. 7 of the drawings. The control mechanism 50 includes a plurality of control members, each of which is in the form of a slide 52. Each petal 14 has a slide 52 associated with it.

To facilitate simultaneous movement of the petals 14 from their first, closed position to their second, open position, the control mechanism 50 includes a connector in the form of a collar 54 receivable over the slides 52 as shown in Fig. 7 of the drawings. However, as shown in Fig. 10 of the drawings, the collar 54 can be removed to facilitate independent manipulation of the slides 52 and, consequently, independent manipulation of the petals 14 of the catheter sheath 10.

The pull wire 28 of each petal 14 is connected to its associated slide 52 as shown in greater detail in Figs. 8 and 9 of the drawings.

In order to effect steering of the catheter sheath 10, the catheter assembly 40 includes a stylet 56 (Fig. 12) received in the primary lumen 18 of the catheter sheath 10. The stylet 56 is a steerable stylet and has an outer, tubular member 58 defining a passage in which a conductive, elongate actuator 60 is received. The actuator 60 is insulated from the tubular member 58 by an insulating sleeve 62.

The stylet 56 supports an end electrode 64 on its distal end. In the illustrated embodiment, the end electrode 64 is a dome electrode and is supplied with energy via the conductive actuator 60. The electrode 64 is mounted on the distal end of the tubular member 58 via a force absorbing element in the form of a bellows-like member 66.

In other embodiments (not illustrated), the end electrode 64 could be a needle electrode for effecting transmural ablation of tissue at a site in a patient's body.

The tubular member 58 has, proximally of its distal end, a bend-enhancing region in the form of a longitudinally extending cut-away portion (not shown). The cut-away portion subtends an angle of approximately 270° to leave a longitudinally extending spine of material. The actuator 60 is mechanically fast with the distal end of the tubular member 58 so that, by relative axial displacement between the tubular member 58 and the actuator 60, bending of the tubular member and, accordingly, the catheter sheath 10 about the bend-enhancing region of the tubular member 58 is effected. Thus, although not illustrated, the actuator 60 has it proximal end fast with the handle 42. The tubular member 58 of the stylet 56 is connected to a steering control mechanism 68 (Figs. 7-10). The steering control mechanism 68 is displaceable axially relative to the remainder of the handle 42 for effecting displacement of the tubular member 58 relative to the actuator 60 to effect steering of the distal end of the catheter sheath 10.

In the illustrated embodiment, the end electrode 64 is fixedly positioned relative to the catheter sheath 10 and, when the petals 14 are in their closed position, they envelop the end electrode 64. When the petals 14 are displaced to their open position, the end electrode 64 lies substantially in the same plane as the electrodes 22 of the petals 14 as shown in Fig. 11 of the drawings.

In another embodiment, the end electrode 64 is mounted distally of the petals 14 when they are in their closed position and the stylet 56 is displaceable relative to the electrode sheath 10 to bring the electrode 64 into the same plane as the electrode 22 of the petals 14 when the petals 14 are in their open position. Thus, the catheter assembly 40 includes a projection control mechanism 70 (Fig. 10) for displacing the catheter sheath 10 distally with respect to the stylet 56 to bring the electrodes 22 and the end electrode 64 into the same plane.

The cables forming the ribbon cable 24 are bundled together into an electrical cable 72 which passes through the handle 42 exiting at the proximal end 44 of the handle 42. In addition, the handle 42 accommodates an irrigation conduit 74 for the transmission of irrigation fluid through the primary lumen 18 to irrigate the site at the patient's body at which ablation is being effected.

The petals 14 are marked with radio opaque marker in order to be able to identify the petals 14. In the illustrated embodiment, the radio opaque markers are a different number of bands 76 (Figs. 1 and 4) on each petal 14. For example, in the present embodiment where four petals 14 are illustrated, one band is provided on a first petal, two bands 76 on a second petal 14, three bands (not shown) on a third petal 14 and four bands (also not shown) on a fourth petal 14. Instead, in another embodiment, the electrodes 22 are formed from a mixture of platinum foil and metal coating with the mixture varying from petal to petal so that the relevant petals 14 and their associated electrodes 22 can be identified. Thus, the electrode 22 associated with the first petal could be a metal coating, a second petal could have a distal 3mm of platinum foil and a proximal 6mm of a different metal coating, the third petal could have an electrode 22 comprising a distal 6mm of platinum foil with a proximal 3mm of a different metal coating and the fourth petal could have an electrode 22 which is all of platinum foil.

The catheter assembly 40 is used in the heat treatment of a site in a patient's body. A particular application of the catheter assembly is ablation treatment in the treatment of heart arrhythmias. The catheter sheath 10, with the petals 14 in their closed position, is fed through the vasculature of the patient to the desired region in the heart at which the heart arrhythmias are to be treated. At the chosen site, the control mechanism 50 is operated to open the petals 14 so that they adopt their second position as shown, for example, in Fig. 11 of the drawings. Use is then made of any two of the electrodes for effecting the desired pattern of ablation at the site. Thus, for example, one of the strip electrodes 22 of one of the petals 14 could be used in conjunction with the dome electrode 64, two opposed strip electrodes 22 could be used or two strip electrodes 22 adjacent each other could be used.

The electrodes 22 are energised with out-of-phase energy as described in greater detail in the Assignee's co-pending International Patent Application No. PCT/AU03/01421 dated 28 October 2003 and entitled "System for, and method of, heating a biological site in a patient's body".

As described in that International Application, the energy supplied to any one electrode is 180° out of phase with the energy supplied to any other electrode. The sum of the energy is, however, still the same and, using this method, longer but shallower legions are formed resulting in less trauma at the treated site in the patient's body. In addition, the shape of the strip electrodes 22 promotes the formation of longer lesions at the site thereby reducing the time taken to complete an ablation procedure.

In the case where the end electrode 64 is a needle electrode, the catheter assembly 10 could be used in the treatment of ventricular tachycardia. To enable tissue to be treated, the needle electrode 72 is carried on the distal end of the stylet 56. Because penetration of the tissue is required, the bellows-like member 66 is omitted. With this arrangement, the needle electrode enters the tissue to be treated and is used in combination with one or more of the strip electrodes 22.

It is therefore an advantage of the invention that a catheter sheath 10 is provided which is able to be manipulated into a configuration where longer, shallower lesions can be formed thereby reducing time for completing an ablation procedure. The use of the petal-like arrangement at the distal end of the catheter sheath 10 enables a larger area of the site to be treated. Further, the use of the out-of-phase energy facilitates treatment of such a larger area. A further advantage is that, because the strip electrodes 22 are long and cover a larger area, reduced manipulation of the catheter sheath 10, while in the patient's body, is required.

As indicated above, the use of long strip electrodes 22 provides for shallow lesions to be formed at the site which reduces trauma but is also more effective in the treatment of heart arrhythmias.

It is yet a further advantage of the invention that the petals 14 are resiliently flexible, either as a result of the plastics material from which they are made or with the addition of the Nitinol strips 32. This promotes tissue-electrode contact and reduces the risk of inadequate contact as a result of surface irregularities.

It will be appreciated by persons skilled in the art that numerous variations and/or modifications may be made to the invention as shown in the specific embodiments without departing from the scope of the invention as broadly described. The present embodiments are, therefore, to be considered in all respects as illustrative and not restrictive.

## Claims

1. A catheter sheath which includes
an elongate element defining a primary lumen and a plurality of secondary lumens arranged in spaced relationship about the primary lumen;
a plurality of discrete members defined by the elongate element, the discrete members being displaceable between a first position extending parallel to a longitudinal axis of the elongate element and a second position in which the discrete members extend transversely to the longitudinal axis of the elongate element;
at least one electrode carried by at least one of the discrete members; and
electrical conductors for the at least one electrode arranged in the primary lumen, the primary lumen being shaped to accommodate the conductors.

2. The catheter sheath of claim 1 in which an inner surface of a wall of the elongate element defining the primary lumen is recessed to define at least one axially extending channel in which the conductors are receivable.

3. The catheter sheath of claim 2 in which each discrete member carries at least one electrode and in which a plurality of circumferentially spaced channels is defined in the inner surface of the wall of the elongate element, each discrete member having a channel associated with it.

4. The catheter sheath of claim 3 in which the at least one electrode of each discrete member is arranged on an operatively inner surface of its associated discrete member.

5. The catheter sheath of any one of the preceding claims in which the discrete members are defined by the elongate element.

6. The catheter sheath of claim 5 in which the discrete members are defined by longitudinally extending slits at least at the distal end of the elongate element.

7. The catheter sheath of any one of the preceding claims in which a control element is received in each secondary lumen, each control element being connected to one of the discrete members and the control element controlling movement of its associated discrete member at least from its first position to its second position.

8. The catheter sheath of any one of the preceding claims which includes an urging means associated with each discrete member for urging the discrete member from its second position to its first position.

9. The catheter sheath of claim 8 in which each urging means is a resiliently flexible component having at least a portion inwardly of a centre plane of its associated discrete member.

10. A catheter assembly which includes
a handle having a proximal end and a distal end;
a catheter sheath as claimed in claim 7 attached to a distal end of the handle; and
a control mechanism carried by the handle, the control elements of the catheter sheath being connected to the control mechanism.

11. The assembly of claim 10 which includes a stylet received in the primary lumen of the elongate element of the catheter sheath.

12. The assembly of claim 11 in which the stylet is a steerable stylet for effecting steering of the catheter sheath, in use, the handle including a steering control mechanism to which the stylet is connected for effecting steering of the stylet.

13. The assembly of claim 10 or claim 11 in which a distal end of the stylet carries an end electrode.

14. The assembly of claim 13 in which the end electrode is a dome electrode.

15. The assembly of claim 13 or claim 14 in which the end electrode is arranged to lie substantially in the same plane as the at least one electrode of the at least one discrete member when that discrete member is in its second position.

16. The assembly of claim 13 or claim 14 in which the stylet and the catheter sheath are axially displaceable with respect to each other so that, when the discrete members of the elongate element of the catheter sheath are in their second position, the end electrode can be brought into substantially the same plane as the at least one electrode of the at least one discrete element.

17. The assembly of claim 13 in which the end electrode is a needle electrode.

18. The assembly of any one of claims 10 to 17 in which the control mechanism includes a plurality of control members, each control member being connected to one of the control elements of the catheter sheath so that movement of each discrete member can be effected independently of the remaining discrete members, the control mechanism further including a connector for interconnecting the control members to effect simultaneous control of the discrete members of the catheter sheath.
